# EUROPEAN PATENT APPLICATION

(11) **EP 4 706 645 A1**
(43) Date of publication of application: **11.03.2026**
(21) Application number: 24800278.4
(22) Date of filing: 03.05.2024
(51) Int. Cl.: A61K 9/51, A61P 25/28

(54) **PEGYLATED RECONSTITUTED HIGH-DENSITY LIPOPROTEIN NANOPARTICLES**

(30) Priority: 04.05.2023 KR 20230058759
(71) Applicant: Mepsgen Co., Ltd., Seoul 05836 (KR)
(72) Inventor: LEE, Wooseung, Seoul 05667 (KR); KIM, Jin, Yongin-si Gyeonggi-do 17095 (KR); KIM, Yong Tae, Seoul 05855 (KR)
(74) Representative: Mullholland, Lewis Paul
(86) International application number: PCT/KR2024/006058
(87) International publication number: WO 2024/228597

(57) **Abstract**

The present invention relates to PEGylated reconstituted high-density lipoprotein nanoparticles having the effect of preventing or treating neurodegenerative diseases. Specifically, the present invention relates to nanoparticles and a method for producing the same, a phospholipid layer of the produced nanoparticles being protected by PEG due to PEG-lipid or a derivative thereof being included in the process of preparing a fluid comprising a hydrophobic material and a fluid comprising a hydrophilic material. The PEGylated nanoparticles have the ability to avoid the rejection mechanism of the immune response in the body while maintaining the existing transport ability across the blood-brain barrier, thereby having excellent stability and exhibiting long-term pharmacological effect due to high circulation ability in the body, and thus can be effectively utilized as a drug or a drug carrier.

## Description

### Technical Field

The present invention relates to PEGylated reconstituted high-density lipoprotein nanoparticles and a method for producing the same. The PEGylated reconstituted high-density lipoprotein nanoparticles according to the present invention have the effect of treating and preventing neurodegenerative diseases based on their excellent stability in the body.

### Background Art

Recently, with the rapid increase in the elderly population and the rise in patients with various neurodegenerative diseases, interest in their treatment and prevention has been growing. Neurodegenerative diseases are diseases that cause various symptoms, such as movement disorders, memory impairment, and cognitive impairment, due to the decline or loss of nerve cell function. Not only in neurological diseases, but also in the brains of normal adults, a large number of nerve cells die every day, and the number of nerve cells dying increases exponentially with aging.

In particular, Alzheimer's disease (AD) is the most common form of dementia and a representative neurodegenerative disease. Alzheimer's disease, the number of which is rapidly increasing as the population ages, is characterized by senile plaques, which are deposits of amyloid-beta (Aβ) in brain tissue, which are produced by sequential cleavage of amyloid precursor protein (APP) by β, γ-secretase, and neurofibrillary tangles caused by hyperphosphorylation of tau protein, a microtubule-associated protein.

Recently, the development of therapeutic agents targeting these pathological features of Alzheimer's disease has been actively pursued. The most common form of therapeutic agents is based on antibodies, and active research is underway to develop effective antibodies targeting the aforementioned targets and optimize their delivery to affected tissues. However, there are ongoing reports that these antibody-based therapeutic agents can cause cerebral edema or intracerebral microhemorrhages, potentially leading to fatal outcomes for patients.

Meanwhile, reconstituted high-density lipoprotein nanoparticles (rHDL) have the advantage of not having the side effects consistently found in antibody-based therapeutic agents because they are nearly identical to substances found in the body. However, when injected into the body, they are taken up by immune cells residing in normal organs (such as the liver, spleen, and lungs) excluding the target organ (the brain) due to the formation of a protein corona by various proteins in the blood. This leads to problems with low circulation capacity and targeting efficiency due to rapid clearance from the bloodstream. In addition, when injected into the body, they have the potential problem of causing side effects due to secondary inflammatory reactions when taken up by the brain or other normal organs due to the adsorption of pro-inflammatory cytokines (IL-1, IL-6, TNF-α, etc.) that are the cause of inflammatory reactions among various types of proteins in the blood.

Therefore, the present inventors developed a composition of PEGylated reconstituted high-density lipoprotein nanoparticles (P-rHDL) that can exhibit excellent therapeutic effects on neurodegenerative diseases and exhibit excellent stability in the body; and a method for producing the same.

### Prior Art Document

(Patent Document 1) Korean Patent Registration No. 10-2531293
(Patent Document 2) Korean Patent Registration No. 10-2631907

(Non-Patent Document 1) Rumiana, T. et al. Lipid nanoparticles-from liposomes to mRNA vaccine delivery, a landscape of research diversity and advancement. ACS Nano 15, 11, 16982-17015 (2021).
(Non-Patent Document 2) Kim, Y. et al. Single step reconstitution of multifunctional high-density lipoprotein-derived nanomaterials using microfluidics. ACS Nano 7, 11, 9975-9983 (2013).

### Detailed Description of Invention

### Technical Problem

An object of the present invention is to provide a PEGylated reconstituted high-density lipoprotein nanoparticle (P-rHDL) having excellent stability in the body.

In addition, another object of the present invention is to provide a method for producing PEGylated reconstituted high-density lipoprotein nanoparticles (P-rHDL).

In addition, another object of the present invention is to provide a composition for preventing or treating neurodegenerative diseases, comprising the PEGylated reconstituted high-density lipoprotein nanoparticles (P-rHDL).

In addition, another object of the present invention is to provide a method for treating neurodegenerative diseases, comprising administering PEGylated reconstituted high-density lipoprotein nanoparticles (P-rHDL) or reconstituted high-density lipoprotein nanoparticles (rHDL) to a patient with a neurodegenerative disease.

### Solution to Problem

The present invention provides a reconstituted high-density lipoprotein (rHDL) nanoparticle comprising a PEGylated lipid (PEG-lipid), a phospholipid, and apolipoprotein E.

In one embodiment, the synthetic mixing molar ratio of the apolipoprotein and the PEGylated lipid is 1:0.5 to 1:50, preferably 1:1 to 1:10, and more preferably 1:1 to 1:5.

In one embodiment, the average molecular weight of the PEG is 550 (0.5k) to 5,000 (5k), and preferably 1,000 (1k) to 2,000 (2k).

In one embodiment, the reconstituted high-density lipoprotein (rHDL) nanoparticle may have a size of 5 to 50 nm, and preferably 10 to 35 nm.

In one embodiment, the PEG in the PEGylated lipid may be PEG or a derivative thereof.

In one embodiment, the apolipoprotein may be apolipoprotein E2 or apolipoprotein E3.

In one embodiment, the reconstituted high-density lipoprotein nanoparticle (rHDL) may further comprise apolipoprotein A1.

In one embodiment, the phospholipid may be at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DCChol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA, but is not limited thereto.

The present invention provides a composition for preventing or treating neurodegenerative diseases, comprising the reconstituted high-density lipoprotein nanoparticles (rHDL), with improved in vivo safety and stability.

In one embodiment, the neurodegenerative disease may be selected from the group consisting of Parkinson's disease, Alzheimer's disease, Pick's disease, Huntington's disease, Creutzfeldt-Jakob disease, Lou Gehrig's disease, spinocerebellar degeneration, spinocerebellar ataxia, prion disease, cognitive dysfunction, senile dementia, dementia with Lewy bodies, frontotemporal dementia, vascular dementia, alcoholic dementia, presenile dementia, Machado-Joseph disease, dystonia, multiple system atrophy, progressive supranuclear palsy, Friedreich's ataxia, temporal lobe epilepsy, and stroke, but is not limited thereto. Preferably, the neurodegenerative disease may be Alzheimer's disease.

The reconstituted high-density lipoprotein nanoparticles (rHDL) maintain specific ligand activity against the blood-brain barrier tissue while simultaneously avoiding phagocytosis by immune cells.

The present invention provides an improved method for treating neurodegenerative diseases, comprising administering the reconstituted high-density lipoprotein nanoparticles with improved in vivo safety and stability to a patient with a neurodegenerative disease.

The present invention provides a method for producing PEGylated reconstituted high-density lipoprotein (P-rHDL), wherein the method comprises: a step of injecting a PEGylated lipid solution into one channel inlet located in the center of a microfluidic device comprising three inlets and one outlet, and a step of injecting high-density lipoprotein (HDL) or reconstituted high-density lipoprotein (rHDL) comprising an apolipoprotein and a phospholipid into two channel inlets located on either side.

The present invention provides a reconstituted high-density lipoprotein nanoparticle (rHDL) produced by the above production method.

### Effects of Invention

The PEGylated reconstituted high-density lipoprotein nanoparticles (P-rHDL) according to the present invention and a composition comprising the same can be used for the prevention or treatment of neurodegenerative diseases.

In addition, the PEGylated reconstituted high-density lipoprotein nanoparticles (P-rHDL) according to the present invention have the effect of avoiding the loss of efficacy of a therapeutic agent due to immune responses in the body through PEGylation, and thus can have excellent effects even at low doses.

### Brief Description of Drawings

Figure 1 is a schematic diagram showing a method for synthesizing PEGylated high-density lipoprotein nanoparticles (P-rHDL) using the reconstituted high-density lipoprotein nanoparticles (rHDL) according to the present invention.
Figure 2 shows the results obtained by measuring P-rHDL by DLS, in which the average molecular weight of the polyethylene glycol (PEG) in the PEGylated lipid is 0.5k, and the synthetic mixing molar ratio of the apolipoprotein and the PEGylated lipid is 1:1.
Figure 3 shows the results obtained by measuring P-rHDL by DLS, in which the average molecular weight of the PEG in the PEGylated lipid is 1k, and the synthetic mixing molar ratio of the apolipoprotein and the PEGylated lipid is 1:1.
Figure 4 shows the results obtained by measuring P-rHDL by DLS, in which the average molecular weight of the PEG in the PEGylated lipid is 2k, and the synthetic mixing molar ratio of the apolipoprotein and the PEGylated lipid is 1:1.
Figure 5 shows the results obtained by measuring P-rHDL by DLS, in which the average molecular weight of the PEG in the PEGylated lipid is 3k, and the synthetic mixing molar ratio of the apolipoprotein and the PEGylated lipid is 1:1.
Figure 6 shows the results obtained by measuring P-rHDL by DLS, in which the average molecular weight of the PEG in the PEGylated lipid is 5k, and the synthetic mixing molar ratio of the apolipoprotein and the PEGylated lipid is 1:1.
Figure 7 shows the results obtained by measuring non-PEGylated rHDL by DLS.
Figure 8 shows the results obtained by measuring P-rHDL by DLS, in which the synthetic mixing molar ratio of the apolipoprotein and the PEGylated lipid (PEG2k-lipid) is 1:0.5, 1:1.
Figure 9 shows the results obtained by measuring P-rHDL by DLS, in which the synthetic mixing molar ratio of the apolipoprotein and the PEGylated lipid (PEG2k-lipid) is 1:5, 1:10.
Figure 10 shows the results obtained by measuring P-rHDL by DLS, in which the synthetic mixing molar ratio of the apolipoprotein and the PEGylated lipid (PEG2k-lipid) is 1:20, 1:50.
Figure 11 shows the results obtained by comparing the apolipoprotein incorporation efficiency of P-rHDL, in which the mixing molar ratio of the rHDL and the PEGylated lipid (PEG2k-lipid) is 1:5, 1:10, and 1:20, respectively.
Figure 12 shows the results obtained by comparing the surface charge of rHDL and P-rHDL based on zeta potential values.
Figure 13 shows the results obtained by comparing the distribution and storage stability of P-rHDL at days 0 and 21 using DLS.
Figure 14 shows the results obtained by confirming the effect of inhibiting amyloid-beta aggregation when treated simultaneously with amyloid-beta and P-rHDL.
Figure 15 shows the results obtained by confirming the effect of degrading aggregated amyloid-beta when treated with P-rHDL after inducing amyloid-beta aggregation.
Figure 16 shows the results obtained by measuring the absorbance spectrum of P-rHDL into which a fluorescence-PEGylated lipid is incorporated, wherein the average molecular weight of the PEG is 1k.
Figure 17 shows the results obtained by measuring the absorbance spectrum of P-rHDL into which a fluorescence-PEGylated lipid is incorporated, wherein the average molecular weight of the PEG is 2k.
Figure 18 shows the results obtained by measuring the absorbance spectrum of P-rHDL into which a fluorescence-PEGylated lipid is incorporated, wherein the average molecular weight of the PEG is 3k.
Figure 19 shows the results obtained by confirming the function of fluorescently labeled rHDL and fluorescently labeled P-rHDL to avoid phagocytosis of immune cells using a confocal laser microscope.
Figure 20 shows the results obtained by confirming the intracellular delivery efficiency of fluorescently labeled rHDL in human brain microvascular endothelial cells (hBMEC) using a confocal laser microscope.
Figure 21 shows the results obtained by confirming the intracellular delivery efficiency of fluorescently labeled P-rHDL in hBMEC using a confocal laser microscope.
Figure 22 shows the results obtained by confirming the intracellular delivery efficiency of fluorescently labeled rHDL and P-rHDL in hBMEC monolayers using a confocal laser microscope.
Figure 23 shows the results of quantitative analysis of the intracellular delivery efficiency of fluorescently labeled rHDL and P-rHDL in hBMEC monolayers based on fluorescence images.
Figure 24 shows the results obtained by confirming the intracellular delivery efficiency of fluorescently labeled rHDL and P-rHDL in stem cell-derived brain microvascular endothelial cell (iBMEC) monolayers using a confocal laser microscope.
Figure 25 shows the results of quantitative analysis of the intracellular delivery efficiency of fluorescently labeled rHDL and P-rHDL in iBMEC monolayers based on fluorescence images.
Figure 26 is a graph comparing the blood-brain barrier (BBB) permeation efficiency of rHDL and P-rHDL for up to 4 hours in a blood-brain barrier (BBB) formed with hBMEC monolayers in a Transwell.
Figure 27 is a graph comparing the blood-brain barrier (BBB) permeation efficiency of rHDL and P-rHDL for up to 24 hours in a blood-brain barrier (BBB) formed with hBMEC monolayers in a Transwell.

### Best Mode for Carrying out the Invention

Hereinafter, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention provides a reconstituted high-density lipoprotein (rHDL) comprising a phospholipid, a PEGylated lipid (PEG-lipid), and an apolipoprotein.

As used herein, the term "reconstituted high-density lipoprotein (rHDL)" refers to artificially produced HDL-mimetic particles designed to mimic the biological effects of naturally occurring high-density lipoprotein (HDL).

As used herein, the term "PEGylated" refers to a substance whose surface has been modified with PEG or a derivative thereof.

As used herein, the term "PEG" or "polyethylene glycol" refers to a linear polymer with a terminal hydroxy group, having the structure H-(OCH₂CH₂)ₙ-OH, and whose terminals can be substituted with functional groups such as carboxylic acid, amine, azide, cyanine, or NHS ester.

As used herein, the PEG may have an average molecular weight of 550 (0.5k) to 5,000 (5k), and preferably 1,000 (1k) to 2,000 (2k).

As used herein, the term "neurodegenerative disease" refers to a disease in which the function of the nervous system declines or is lost due to degenerative changes in nerve cells. Preferably, the neurodegenerative disease may be at least one selected from the group consisting of Parkinson's disease, Alzheimer's disease, Pick's disease, Huntington's disease, Creutzfeldt-Jakob disease, Lou Gehrig's disease, spinocerebellar degeneration, spinocerebellar ataxia, prion disease, cognitive dysfunction, senile dementia, dementia with Lewy bodies, frontotemporal dementia, vascular dementia, alcoholic dementia, presenile dementia, Machado-Joseph disease, dystonia, multiple system atrophy, progressive supranuclear palsy, Friedreich's ataxia, temporal lobe epilepsy, and stroke. More preferably, the neurodegenerative disease may be Alzheimer's disease.

The term "apolipoprotein E" refers to a mammalian protein encoded by the APOE gene or a functional variant thereof. In a preferred embodiment, the apolipoprotein E is a human protein encoded by the human APOE gene on chromosome 19. The apolipoprotein E may be any one of the isoforms of the APOE gene product, such as apolipoprotein E2 ("APOE2"), apolipoprotein E3 ("APOE3"), and apolipoprotein E4 (APOE4). A "functional variant" thereof refers to a variant of a mammalian protein encoded by the APOE gene that retains the same or similar biological function as the APOE gene product. In some cases, the functional variant comprises amino acid insertions, deletions, and/or substitutions compared to the protein encoded by the human APOE gene. In some cases, the functional variant is a fragment of the protein encoded by the human APOE gene.

In some embodiments, the apolipoprotein E2 is a protein disclosed in GenBank under the accession number ARQ79459 or a protein having at least 95% sequence identity thereto, preferably at least 98% or 99% sequence identity thereto. In some embodiments, the apolipoprotein E3 is a protein disclosed in GenBank under the accession number ARQ79461.1 or a protein having at least 95% sequence identity thereto, preferably at least 98% or 99% sequence identity thereto.

In some embodiments, the apolipoprotein E in the reconstituted high-density lipoprotein (rHDL) is a recombinant protein produced by genetic engineering, or a synthetic protein produced by chemical synthesis.

The term "apolipoprotein A1" refers to a mammalian protein encoded by the APOA1 gene or a functional variant thereof. In a preferred embodiment, the apolipoprotein A1 is a human protein encoded by the human APOA1 gene located on chromosome 11. A "functional variant" thereof refers to a variant of a mammalian protein encoded by the APOA1 gene that retains the same or similar biological function as the APOA1 gene product. In some cases, the functional variant comprises amino acid insertions, deletions, and/or substitutions compared to the protein encoded by the human APOA1 gene. In some cases, the functional variant is a fragment of the protein encoded by the human APOA1 gene.

As used herein, the "phospholipid" may be specifically at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DCChol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA, but is not limited thereto.

As used herein, the "PEG-lipid" may be PEG or a derivative thereof. Specifically, it may be at least one selected from the group consisting of 1,2-distearoyl-sn-glycero-3-phosphoethanolamine-N-[methoxy(polyethylene glycol)] (PEG PE), 1,2-dimyristoyl-rac-glycerol methoxypolyethylene glycol (DMG-PEG), distearoyl-rac-glycerol methoxypolyethylene glycol (DSG-PEG), polyethylene glycol ceramide (PEG-ceramide), polyethylene glycol-phosphatidylethanolamine (PEG-PE), dodecyl-polyethylene glycol-carboxylic acid (C12-PEG-COOH), octadecyl-polyethylene glycol-carboxylic acid (C18-PEG-COOH), cholesterol-polyethylene glycol-acid (cholesterol-PEG-acid), cholesterol-polyethylene glycol-amine (cholesterol-PEG-amine), cholesterol-polyethylene glycol-azide (cholesterol-PEG-azide), cholesterol-polyethylene glycol-dibenzocyclooctyne (cholesterol-PEG-DBCO), cholesterol-polyethylene glycol-fluorescein isothiocyanate (cholesterol-PEG-FITC), cholesterol-polyethylene glycol-maleimide (cholesterol-PEG-maleimide), cholesterol-polyethylene glycol-N-hydroxysuccinimide (cholesterol-PEG-NHS), 1,2-distearoyl-sn-glycero-3-phosphoethanolamine-N-[biotinyl(polyethylene glycol)] (DSPE-PEG-biotin), 1,2-distearoyl-sn-glycero-3-phosphoethanolamine-N-[carboxy(polyethylene glycol)] (DSPE-PEG-COOH), 1,2-distearoyl-sn-glycero-3-phosphoethanolamine-N-[fluorescein isothiocyanate (polyethylene glycol)] (DSPE-PEG-FITC), 1,2-distearoyl-sn-glycero-3-phosphoethanolamine-N-[maleimide(polyethylene glycol)] (DSPE-PEG-maleimide), 1,2-distearoyl-sn-glycero-3-phosphoethanolamine-N-[azido(polyethylene glycol)] (DSPE-PEG-azide), 1,2-distearoyl-sn-glycero-3-phosphoethanolamine-N-[PDP(polyethylene glycol)] (DSPE-PEG-PDP), 1,2-distearoyl-sn-glycero-3-phosphoethanolamine-N-[amino(polyethylene glycol)] (DSPE-PEG-NH2), 1,2-distearoyl-sn-glycero-3-phosphoethanolamine-N-[succinimidyl(polyethylene glycol)] (DSPE-PEG-NHS), N-palmitoyl-sphingosine-1-{succinyl[methoxy(polyethylene glycol)]} (C16-PEG-ceramide), and the like, but is not limited thereto.

### [Example 1]

### Method for producing PEGylated reconstituted high-density lipoprotein nanoparticles (P-rHDL)

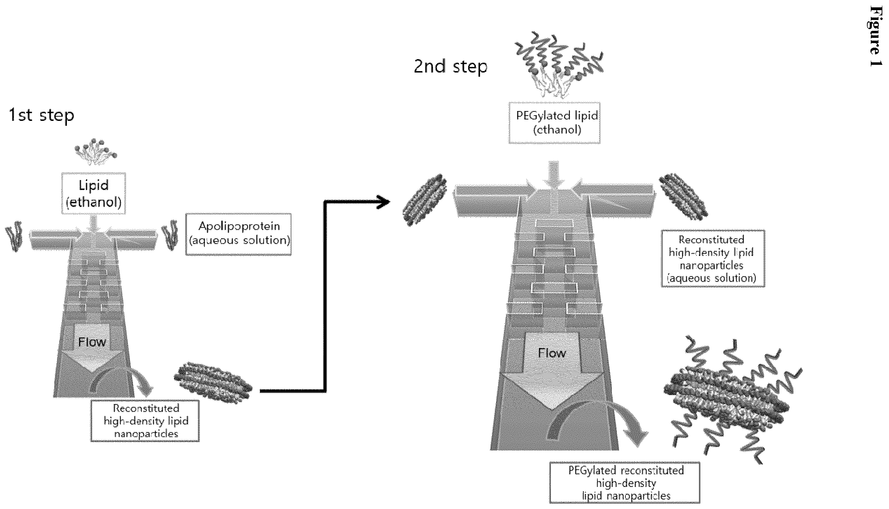

In order to produce PEGylated reconstituted high-density lipoprotein nanoparticles (PEGylated recombinant high density lipoprotein, P-rHDL), as shown in Figure 1, a microfluidic device comprising three inlets and one outlet and having micropillars inside the device was used to produce high-density lipoprotein nanoparticles (rHDL). Thereafter, PEGylated lipids were added to the rHDL to produce P-rHDL.

### 1-1 Method for producing P-rHDL using a microfluidic device 1

First, in order to produce rHDL, DMPC (1,2-dimyristoyl-sn-glycero-3-phosphocholine), a phospholipid, was prepared in absolute ethanol at a concentration of 0.83 mg/mL, and apolipoprotein was prepared in PBS or physiological saline at a concentration of 0.2 mg/mL.

About 1 mL of the DMPC solution was injected into an inlet located in the center of a microfluidic device comprising three inlets, and 5 mL of the apolipoprotein solution was injected into the two channels located on either side. The injection flow rate of the DMPC solution was set to 0.8 mL/min and the injection flow rate of the apolipoprotein solution was set to 4.4 mL/min. The two solutions were efficiently mixed by the micropillars inside the device, and rHDL was ultimately obtained through the efflux channel of the micro-mixing channel device. The obtained rHDL was purified using a 10K Amicon filter at 3,900 rpm for 20 minutes and prepared in 2.5 mL of PBS or physiological saline for the next process.

Thereafter, In order to PEGylate rHDL, 500 µL of PEGylated lipid PEG2k-lipid (18:0 PEG2000 PE) was prepared at a concentration of 0.82 mg/mL. The injection flow rate of the PEGylated lipid PEG2k-lipid solution was set to 0.8 mL/min and the injection flow rate of the rHDL solution was set to 4.4 mL/min. Thereafter, it was connected to a new microfluidic device, and P-rHDL was synthesized. P-rHDL obtained through the efflux channel of the microfluidic device was mixed with PBS or physiological saline and then purified three times, once for 20 minutes and twice for 15 minutes, using a new 10K filter at the maximum centrifuge speed at 25 °C. The final purified residue was diluted with physiological saline to a total volume of 1 mL and then filtered through a 0.2-µm syringe filter to obtain the final purified P-rHDL. The produced P-rHDL was stored at 4 °C until use in the next experiment.

### 1-2 Method for producing P-rHDL using a microfluidic device 2

Another method for producing P-rHDL was as follows. The phospholipid DMPC and the PEGylated lipid used for producing rHDL were prepared as a single solution and injected into an inlet located in the center of a microfluidic device, and the apolipoprotein solution was injected into the two channels located on either side.

In detail, a DMPC (1,2-dimyristoyl-sn-glycero-3-phosphocholine) solution and a PEG solution were prepared in absolute ethanol. First, a DMPC solution was prepared at a concentration of 8.3 mg/mL in absolute ethanol. Next, a PEG2k-lipid solution (18:0 PEG2000 PE, a molar ratio of the apolipoprotein and the PEGylated lipid of 1) was prepared at a concentration of 0.82 mg/mL in absolute ethanol. The PEG2k-lipid solution was thoroughly dispersed using a bath sonicator for about 30 minutes, which was confirmed visually.

100 µL of the DMPC solution, 100 µL of the PEG2k-lipid solution, and 800 µL of absolute ethanol were mixed, and then the mixture was homogenized for about 10 minutes using a digital rocker to produce a DMPC/PEG2k-lipid solution.

Thereafter, P-rHDL comprising a phospholipid (DMPC), PEG, and an apolipoprotein was produced in the same manner as in Example 1-1 above.

### 1-3 Optimization of PEG molecular weight in PEGylated lipid during P-rHDL production

In order to confirm the effect of the average molecular weight of the PEG in the PEGylated lipid on the production of P-rHDL, the presence or absence of P-rHDL synthesis was confirmed according to the molecular weight of the PEG in the PEGylated lipid. The P-rHDL synthesis method was the same as in Example 1-2 above, and The PEGylated lipid, in which the molecular weight of the PEG in the PEGylated lipid is 0.5, 1, 2, 3, 5k, was used. At this time, the molar ratio of the apolipoprotein and the PEGylated lipid was 1:1.

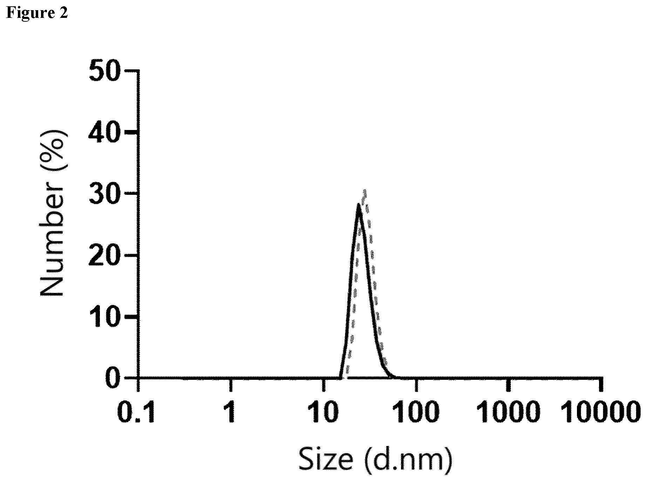

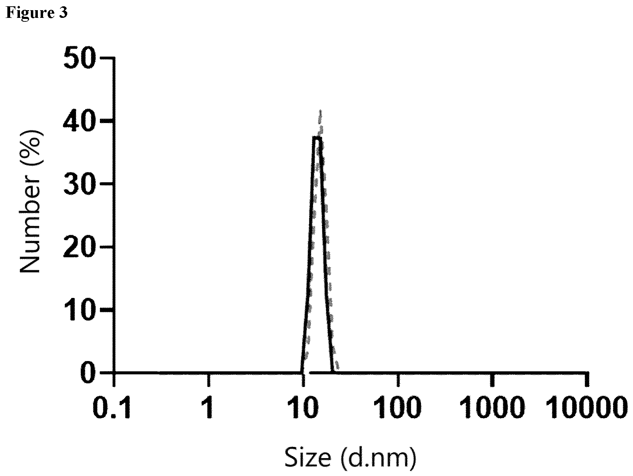

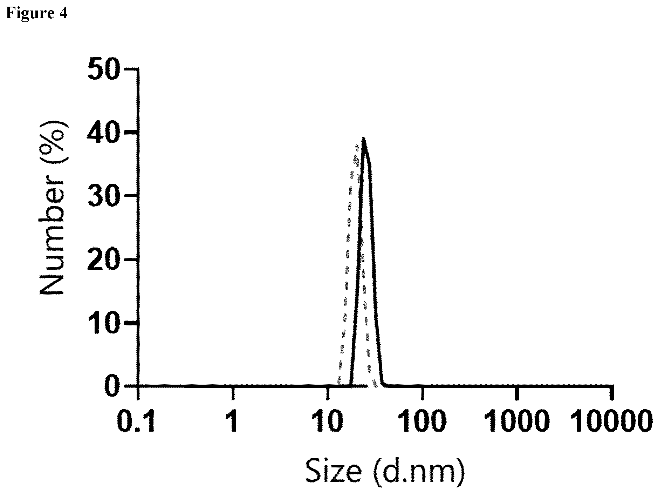

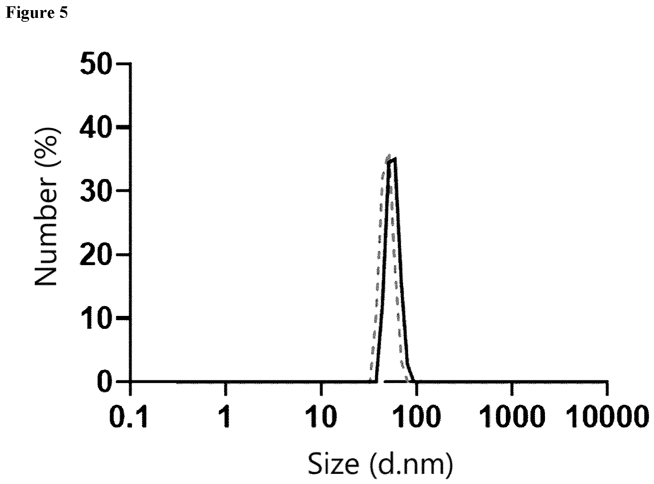

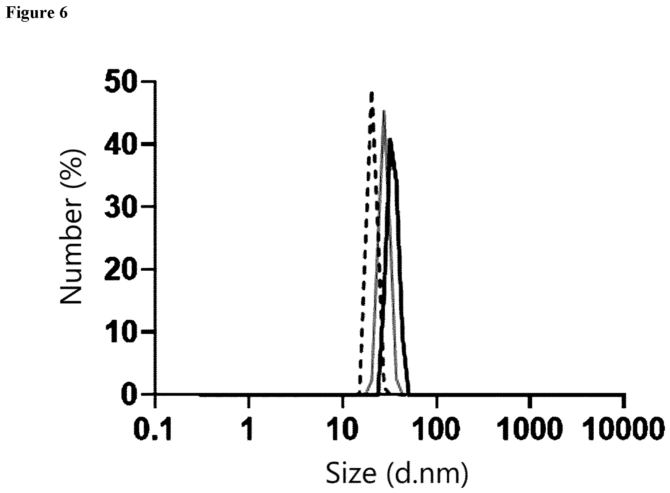

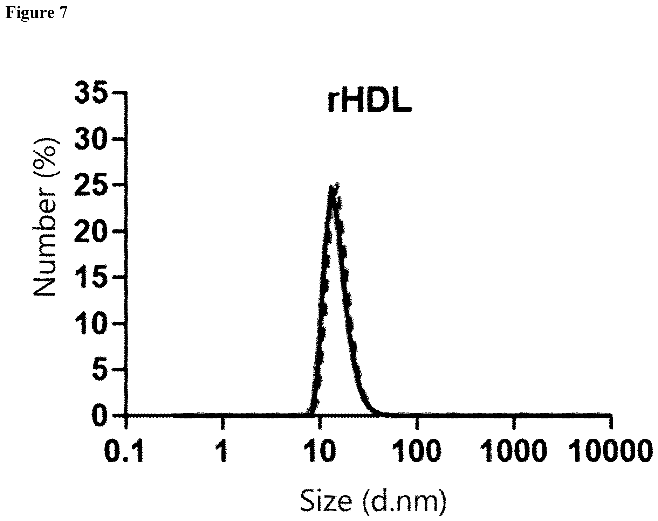

P-rHDL synthesized under the above conditions was diluted 10-fold with PBS, and then the size distribution diagram using dynamic light scattering (DLS) is shown in Figures 2 to 6. Based on the above, it was confirmed that P-rHDL was synthesized regardless of the PEG molecular weight. In particular, it was confirmed through Figures 3 and 4 that the molecular weight of PEG, which has a similar graph shape to the DLS results of rHDL in Figure 7, i.e., a size similar to that of rHDL, is 1k or 2k.

### 1-4 Optimization of PEGylated lipid mixing ratio during P-rHDL production

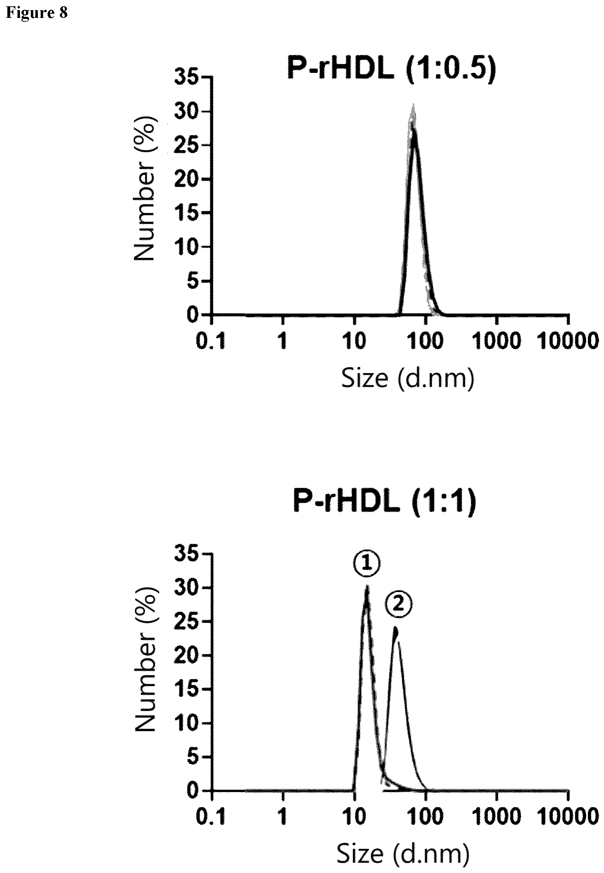

As shown in Figures 8, 9, and 10, in order to optimize the mixing ratio of the PEGylated lipid in P-rHDL, P-rHDLwas produced in the same manner as in Examples 1-2 and 1-3. At this time, the mixing ratio of apolipoprotein ApoE3 and PEGylated lipid PEG2k-lipid was varied (1:0, 1:0.5, 1:1, 1:5, 1:10, 1:20, 1:50), and the sizes of the produced P-rHDL were compared using DLS.

**[Table 1]**

| P-rHDL synthesis conditions according to the mixing ratio of PEG2k-lipid | | | | |
|---|---|---|---|---|
| Nanoparticle | ApoE3: PEG2k-lipid (molar ratio) | PEG2k-lipid moles (nmol) | PEG2k-lipid amount (mg) | PEG2k-lipid (mol%) |
| rHDL | 1:0 | 0 | 0 | 0 |
| P-rHDL | 1:0.5 | 14.6 | 0.04 | 2.2 |
| | 1:1 | 29.1 | 0.08 | 4.3 |
| | 1:5 | 145 | 0.41 | 18.2 |
| | 1:10 | 291 | 0.81 | 30.8 |
| | 1:20 | 581 | 1.63 | 47.1 |
| | 1:50 | 1450 | 4.07 | 69.0 |

As shown in Table 1 above, P-rHDL was produced in the same manner as in Example 1-1 according to each mixing ratio of PEG2k-lipid based on apolipoprotein.

**[Table 2]**

| Comparison of P-rHDL synthesis and property analysis according to the mixing ratio of PEG2k-lipid | | | |
|---|---|---|---|
| Nanoparticles | ApoE3: PEG2k-lipid (molar ratio) | Size (nm) | PDI |
| rHDL | 1:0 | 15.60 | 0.24 |
| P-rHDL | 1:0.5 | 72.48 | 0.41 |
| | 1:1 | 31.44 [① 16.68 nm, ② 46.20 nm] | 0.18 |
| | 1:5 | 16.39 | 0.24 |
| | 1:10 | 18.19 | 0.20 |
| | 1:20 | 11.32 | 0.25 |
| | 1:50 | 32.71 [① 18.67 nm, ② 46.76 nm] | 0.17 |

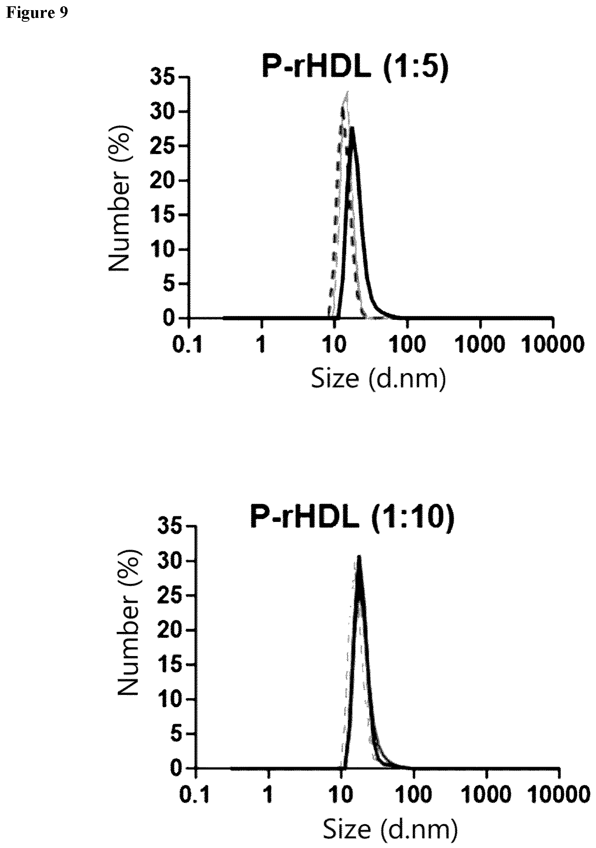

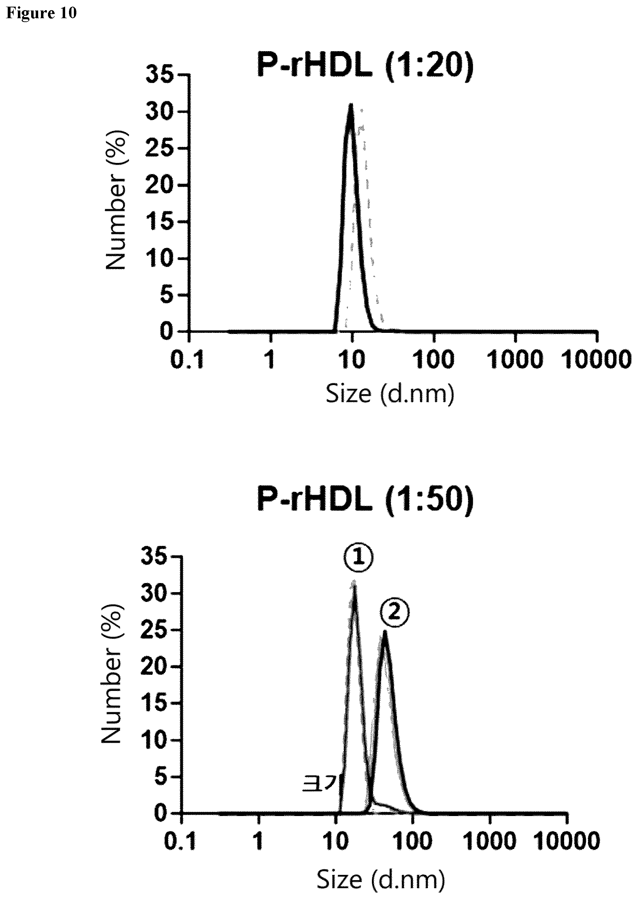

As shown in Table 2 above and Figures 8, 9, and 10, the size and uniformity of the synthesized P-rHDL according to each mixing ratio were confirmed through DLS measurements. At 1:0.5, the synthesized P-rHDL had a size of 70 nm and a PDI of 0.4, showing a very different pattern from rHDL. Except for the 1:0.5 ratio, the PDI was similar to that of rHDL at all mixing ratios (1:1, 1:5, 1:10, 1:20, and 1:50). Compared to the non-PEGylated rHDL, the particle sizes of P-rHDL at mixing ratios of 1:1 and 1:50 were approximately twice as large, at 31.44 and 32.71 nm, respectively. In addition, as shown in Figures 8 and 10, From the number percent (based on number distribution) graph of the P-rDHL DLS results, it was confirmed that, unlike rHDL, two types of particles were generated, resulting in non-uniform particle size distribution. It was confirmed that at mixing ratios of 1:5, 1:10, and 1:20, the particle sizes were 16.39, 18.19, and 11.32 nm, respectively, similar to those of rHDL, and the DLS distribution diagrams in Figures 9 and 10 and the PDI values in Table 2 were similar. In other words, it was confirmed that the ideal conditions for P-rHDL according to the mixing ratio of PEG2k-lipid were mixing ratios of 1:5, 1:10, and 1:20.

In order to confirm the apolipoprotein E3 (ApoE3) incorporation efficiency of P-rHDL synthesized at various mixing ratios (1:5, 1:10, and 1:20), ApoE3 was quantitatively analyzed using the BCA (bicinchoninic acid) assay.

**[Table 3]**

| ApoE3 quantitative results according to the mixing ratio of PEG2k-lipid | | | | | |
|---|---|---|---|---|---|
| Nanoparticles | rHDL | P-rHDL | | | |
| ApoE3:PEG2k-lipid (Molar ratio) | 1:0 | 1:5 | 1:10 | 1:20 | |
| ApoE3 | Feeding amount (µg) | 1000 | | | |
| | Incorporation amount (µg) | 709.6 | 684.9 | 749.0 | 754.1 |
| | Incorporation efficiency (%) | 71.0 | 68.5 | 74.9 | 75.4 |

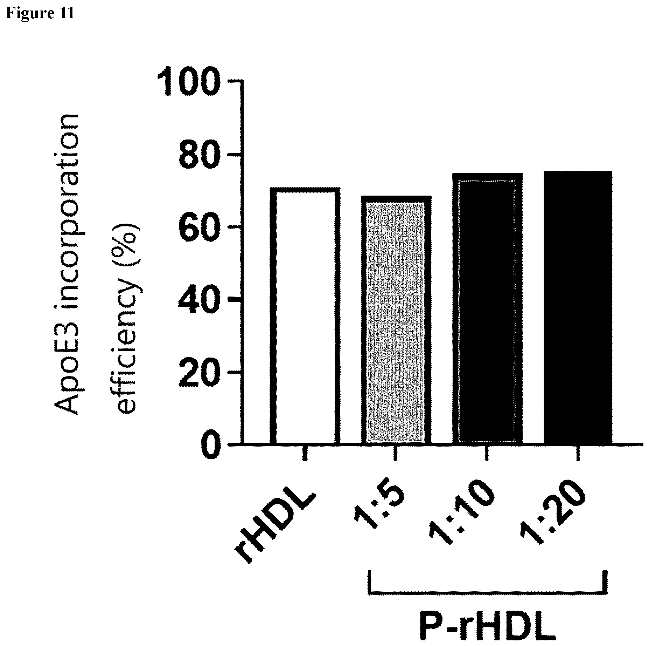

As shown in Table 3 above and Figure 11, it was confirmed that at the mixing ratios of PEG2k-lipid of 1:5, 1:10, and 1:20, the ApoE3 incorporation efficiency was similar to that of non-PEGylated rHDL.

Among the three mixing ratios (1:5, 1:10, and 1:20) of ApoE3 and PEG2k-lipid that synthesize P-rHDL with uniform size, the mixing ratio of 1:5, which allowed for the least incorporation of PEG, an exogenous substance, was selected as the most suitable condition for long-term repeated in vivo administration and pharmaceutical production. Therefore, the 1:5 mixing ratio was used for subsequent validation experiments to produce P-rHDL.

**[Table 4]**

| Measurement of surface charge of P-rHDL | | |
|---|---|---|
| Nanoparticles | Size (nm) | Zeta Potential (mV) |
| rHDL | 15.60 | -6.99 |
| P-rHDL | 16.39 | -7.43 |

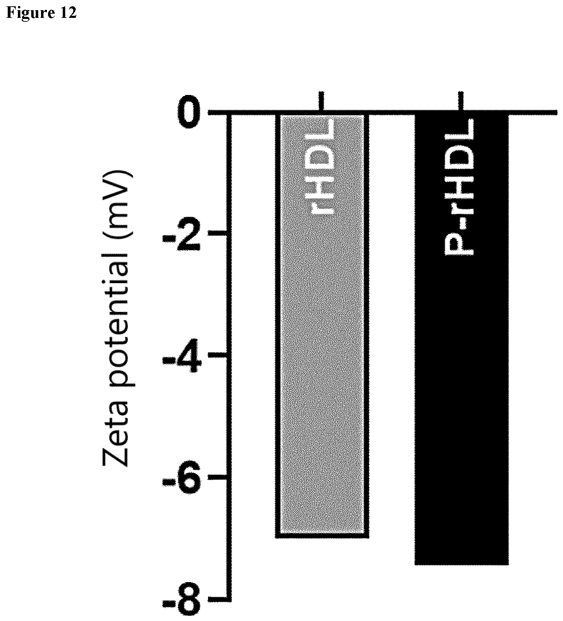

As shown in Table 4 above and Figure 12, it was confirmed that the surface charge of P-rHDL was similar to that of rHDL. In other words, it can be seen that the presence or absence of PEG incorporated did not affect the surface charge. This suggests that PEG does not affect the efficacy of P-rHDL and that it may exhibit similar efficacy to rHDL.

### [Example 2]

### Verification of Stability of P-rHDL

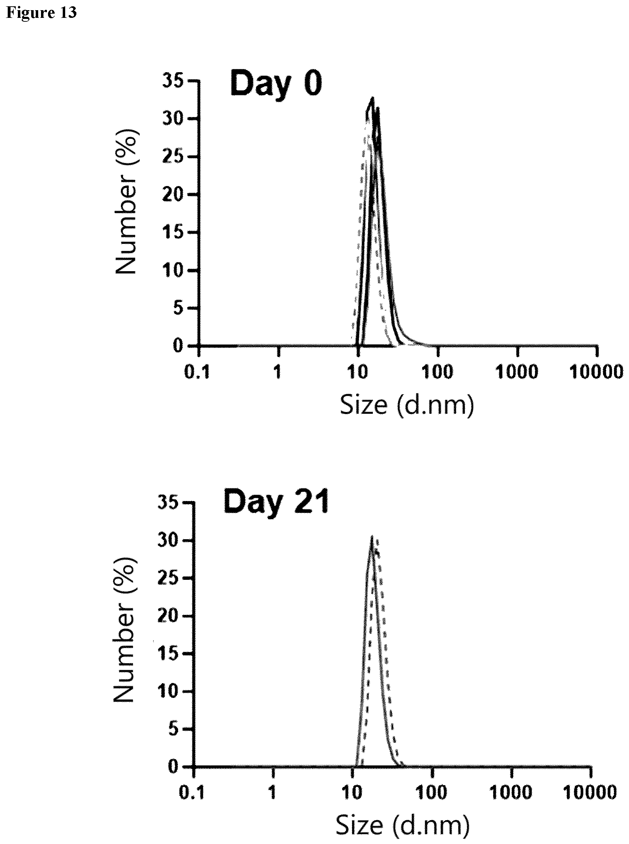

As shown in Table 5 below and Figure 13, immediately after the synthesis of P-rHDL under optimal conditions (0 d), the results of DLS measurements confirmed that P-rHDL had a size of 16.39 nm and a PDI of 0.24, which were similar to those of rHDL. In addition, it was confirmed that P-rHDL, stored at 2 to 8 °C for 21 days to maintain the stability of apolipoprotein and P-rHDL, also had a size of 20.06 nm and a PDI of 0.31. This indicates that the uniformly synthesized P-rHDL has storage stability that can maintain a size similar to that immediately after synthesis for at least 21 days without aggregation between nanoparticles during storage, i.e., without nanoparticles of non-uniform size greater than 50 nm.

**[Table 5]**

| Stability results according to the mixing ratio of PEG2k-lipid | | | | | | |
|---|---|---|---|---|---|---|
| Nanoparticles | ApoE3:PEG2k-lipid (molar ratio) | PEG2k-lipid amount (mg) | PEG2k-lipid (mol%) | Measurement date (day) | Size (nm) | PDI |
| rHDL | 1:0 | 0 | 0 | 0 | 15.60 | 0.24 |
| P-rHDL | 1:5 | 0.41 | 18.2 | 0 | 16.39 | 0.24 |
| | | | | 21 | 20.06 | 0.31 |

### [Example 3]

### Verification of amyloid-beta (Aβ) degradation ability of P-rHDL with or without PEGylation

In order to confirm the possibility of the removal effect of the pathogenic substance Aβ using fluorescently labeled amyloid-beta (Aβ, beta-amyloid(1-42), Hityte^{™} Fluor 488-labeled), the Aβ aggregation inhibition and degradation abilities of rHDL and P-rHDL were compared. Since Aβ has the property of forming plaques by aggregating with each other, which is the main cause of the progression of Alzheimer's dementia, when Aβ aggregates, the fluorescent molecules labeled with Aβ become closer to each other, causing a self-quenching effect, which in turn reduces the fluorescence intensity. Based on this, the aggregation inhibition or degradation abilities of P-rHDL was confirmed.

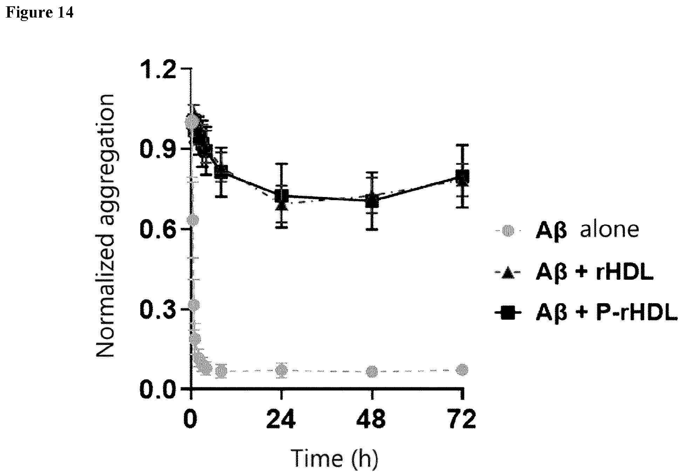

In order to confirm the inhibitory effect of Aβ aggregation, simultaneous treatment with Aβ and P-rHDL and the control group, rHDL, was performed, and the fluorescence intensity was measured at time points (0, 0.25, 0.5, 0.75, 1, 2, 3, 4, 8, 24, 48, and 72 hours) using a microplate reader. As shown in Figure 14, in the group treated with P-rHDL and Aβ, the decrease in Aβ fluorescence was approximately 7 times lower than in the group treated with Aβ alone. Therefore, it was confirmed that P-rHDL exhibited the effect of inhibiting Aβ aggregation. In addition, the degree of fluorescence reduction of Aβ was similar to the control group (rHDL). Therefore, it was confirmed that the two nanoparticles exhibited almost identical inhibitory effects on pathogenic substance aggregation.

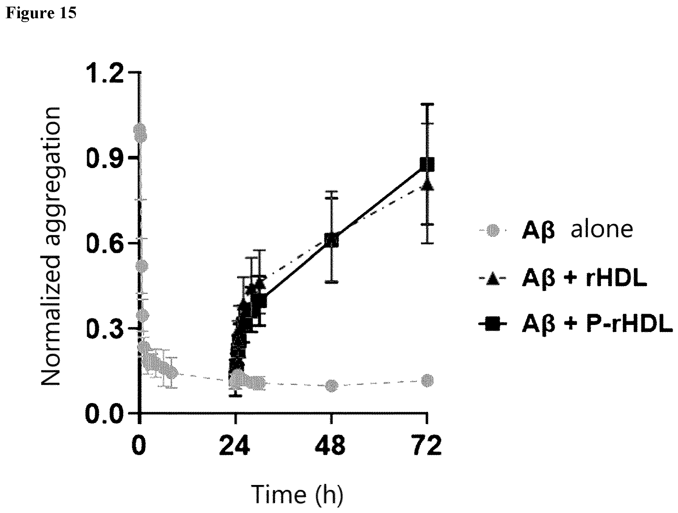

In addition, as shown in Figure 15, in order to confirm the effect of degrading aggregated Aβ, Aβ aggregation was induced for up to 24 hours. At the 24-hour aggregation induction point, it was treated with P-rHDL and rHDL, respectively, and the fluorescence intensity was measured at time points (24, 24.33, 24.67, 25, 26, 28, 30, 48, and 72 hours) using a microplate reader. The fluorescence intensity of Aβ was increased immediately after treatment of aggregated Aβ with P-rHDL. Therefore, it was confirmed that the aggregated Aβ was degraded into peptide form by P-rHDL. In addition, the degree of fluorescence increase of Aβ after treatment was similar to the control group (rHDL). Therefore, it was confirmed that the two nanoparticles exhibited almost identical effect of degrading Aβ.

### [Example 4]

### Verification of PEGylation effect of P-rHDL through evasion of immune cell phagocytosis

### 4-1 Preparation of fluorescence (fluorescein isothiocyanate (FITC)) labeled P-rHDL

In order to verify the PEGylation effect through fluorescence, fluorescently labeled P-rHDL was produced using PEG-lipid labeled with fluorescein isothiocyanate (FITC).

The synthesis of FITC-labeled P-rHDL was performed in the same manner as in Example 1-2, except that FITC-PEG-lipid with PEG molecular weights of 1k, 2k, and 3k were used as the PEGylated lipid, and the molar ratio of apolipoprotein and PEGylated lipid was set to 1:5.

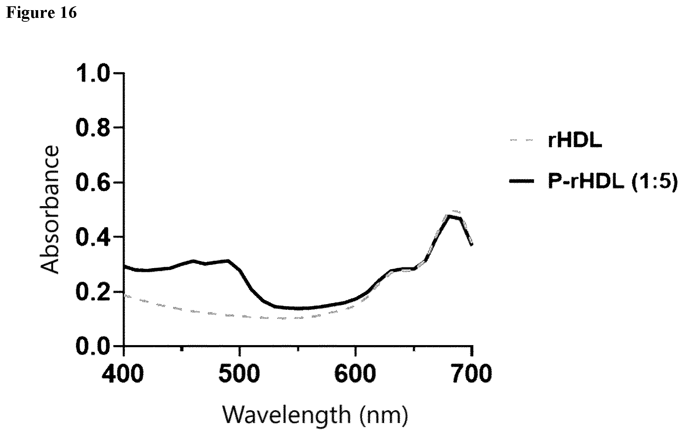

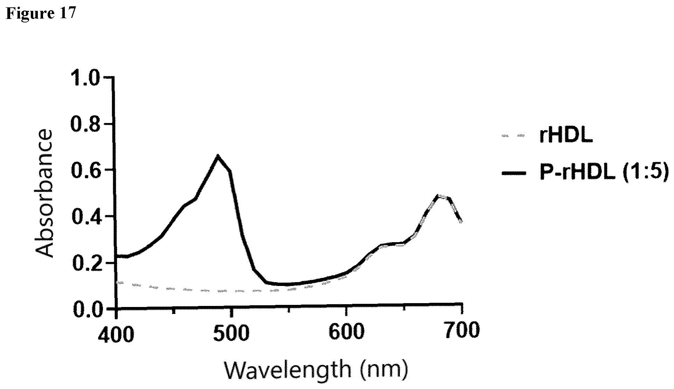

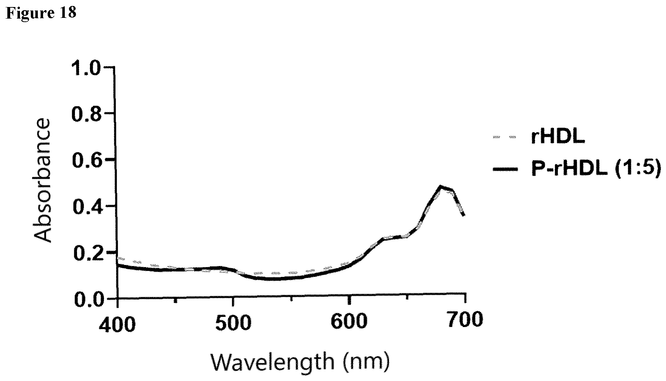

As shown in Figures 16 and 17, when P-rHDL was produced using FITC-PEG with PEG average molecular weights of 1k and 2k, a similar spectrum peak was observed at 490 nm, which is known as the absorbance spectrum peak of the fluorescent substance FITC. As shown in Figure 18, when P-rHDL was produced using FITC-PEG with PEG average molecular weight of 3k, no FITC absorbance spectrum peak was observed at 490 nm.

That is, it can be seen that while P-rHDL can be synthesized when the average molecular weight of PEG is between 0.5k and 5k, the preferred range is between 1k and 2k.

### 4-2 Verification of PEGylation effect of P-rHDL through evasion of immune cell phagocytosis

In order to verify the immune evasion ability of P-rHDL, fluorescently labeled P-rHDL labeled with cyanine5.5 N-hydroxysuccinimide ester (Cy5.5-NHS) was synthesized using the method described in Example 1-2 above. In order to confirm the synthesis of fluorescently labeled P-rHDL, the absorbance spectrum at 665 nm was measured using a microplate reader to analyze Cy5.5 absorbance.

Thereafter, the synthesized fluorescently labeled P-rHDL was tested for phagocytosis by immune cells, verifying the immune evasion ability of P-rHDL. THP-1 cells, used as immune cells, were differentiated into macrophages by treating them with phorbol 12-myristate 13-acetate (PMA) at a concentration of 100 ng/mL for 24 hours. The differentiated THP-1 cells were treated with identical concentrations of fluorescently labeled rHDL and fluorescently labeled P-rHDL. Thereafter, the rHDL and P-rHDL that were influxed into the cells by phagocytosis were observed using a confocal laser scanning microscope (CLSM).

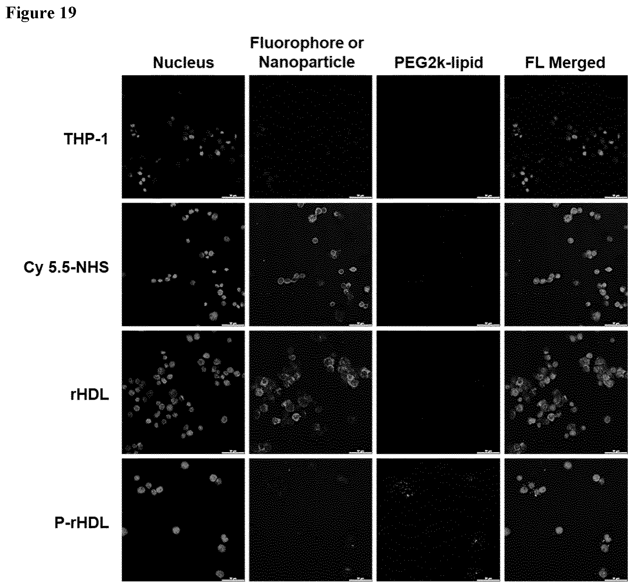

As shown in Figure 19, it was confirmed that P-rHDL showed lower phagocytosis by THP-1 cells compared to non-PEGylated rHDL. This suggests that, as mentioned in the cited literature, the incorporation of PEG functional groups on the particle surface allows P-rHDL to evade phagocytosis by immune cells.

### [Example 5]

### Confirmation of P-rHDL delivery efficiency into brain microvascular endothelial cells

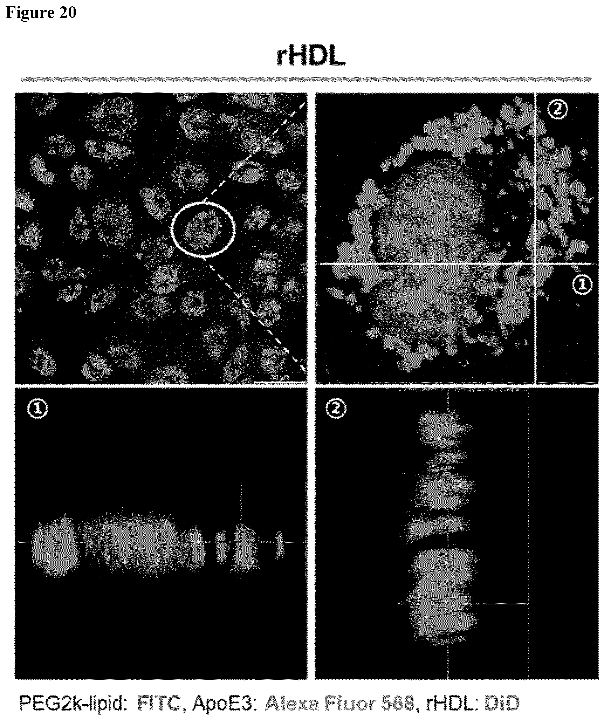

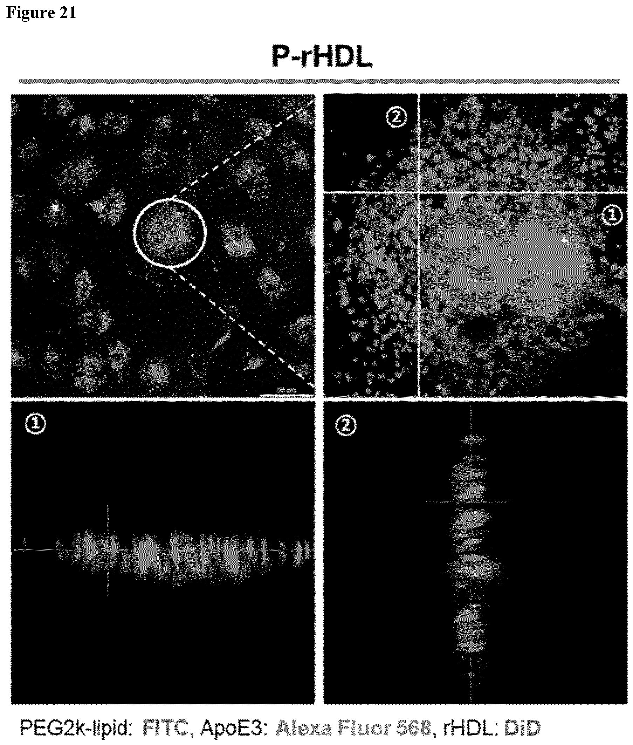

The intracellular delivery efficiency of P-rHDL was confirmed using brain microvascular endothelial cells (BMEC), the major cells forming the blood-brain barrier (BBB). In order to confirm the cell delivery efficiency, among the components of P-rHDL, PEG2k-lipid, ApoE3, and rHDL were labeled with FITC, Alexa Fluor 568, and 1,1'-dioctadecyl-3,3,3',3'-tetramethylindodicarbocyanine, 4-chlorobenzenesulfonate salt (DiD) fluorescent molecules, respectively. For the control group (rHDL), ApoE3 and DMPC were labeled with Alexa Fluor 568 and DiD fluorescent molecules, respectively. In order to confirm the extent of delivery to human-derived brain microvascular endothelial cells (human BMECs, hBMECs), the cells were treated with P-rHDL and rHDL, respectively, for 4 hours at 37°C and 5% CO₂. Thereafter, the cells were fixed, stained for nuclei and cytoplasm, and observed using a confocal laser scanning microscope (CLSM). As shown in Figures 20 and 21, it was confirmed that P-rHDL was delivered around the cell nucleus similarly to the control group rHDL.

In addition, in order to confirm that P-rHDL was delivered intracellularly as a single nanoparticle without degradation, z-stack images of P-rHDL and rHDL uptaken into hBMECs were acquired using CLSM. When examining two different cross-sectional images of hBMEC using z-stack imaging, the intracellularly delivered components of P-rHDL (PEG2k-lipid, ApoE3, and DMPC) were all taken up around the cell nucleus and overlapped at a single location, as shown in Figures 20 and 21. The degree of intracellular delivery around the nucleus and the degree of overlap of the nanoparticle components were similar to those of the control group, rHDL. This suggests that P-rHDL, despite being PEGylated, can still achieve functionally equivalent intracellular delivery efficiency to rHDL.

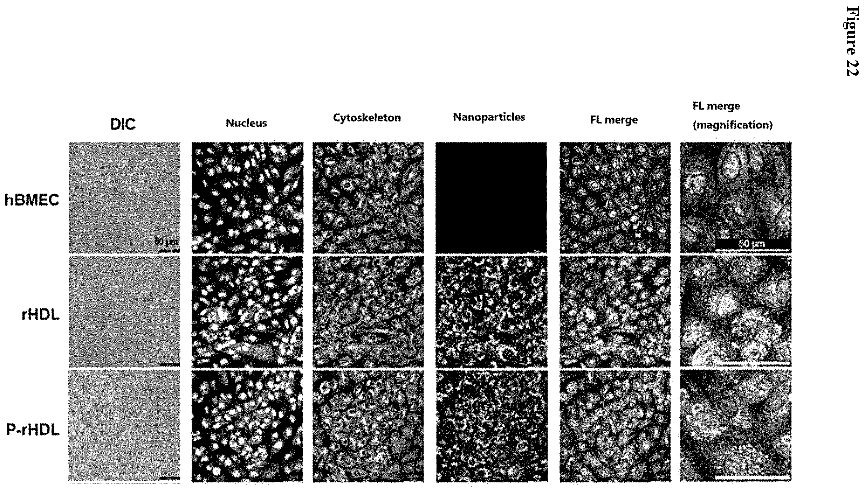

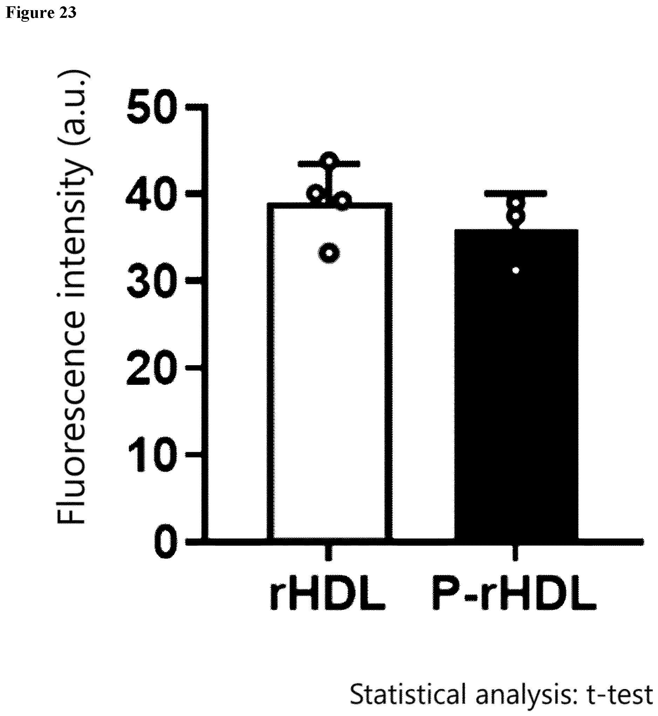

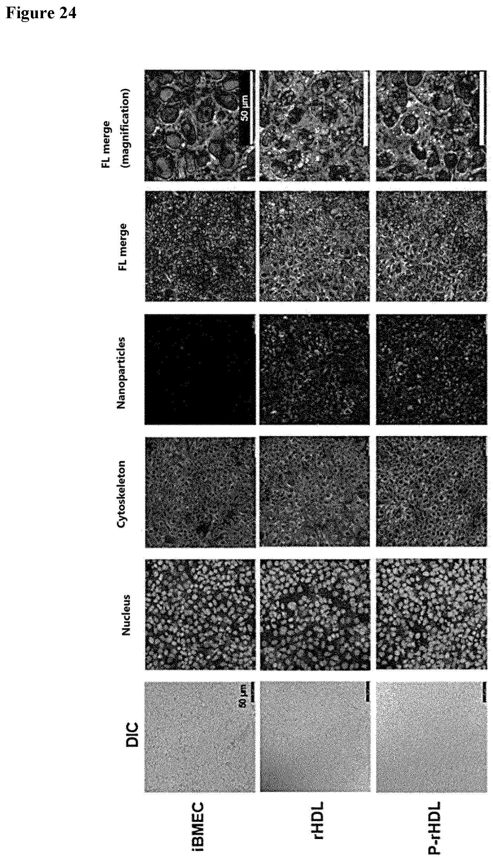

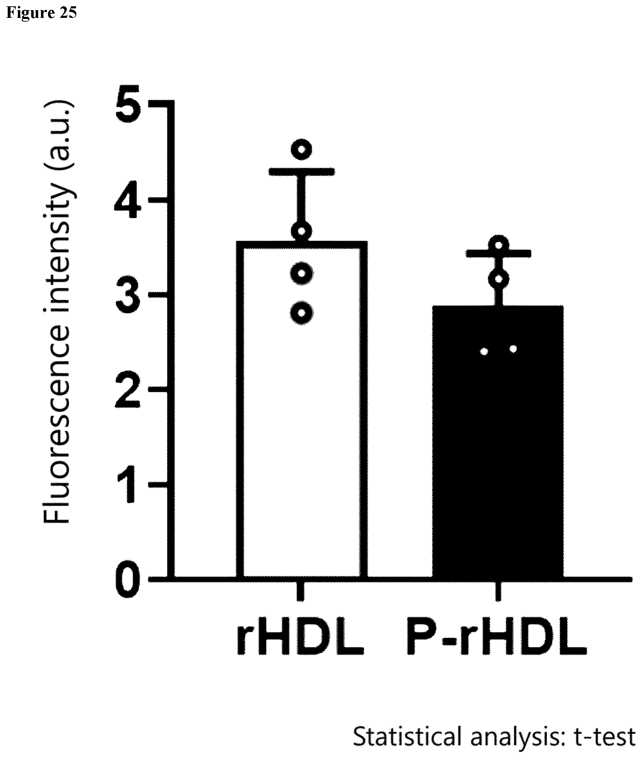

As shown in Figures 22 and 24, human-derived brain microvascular endothelial cells (hBMECs) and induced pluripotent stem cell-derived brain microvascular endothelial cells (iBMECs) were cultured to form a monolayer similar to the in vivo blood-brain barrier, and the intracellular delivery efficiency of P-rHDL was compared with that of the control group, rHDL. As shown in Figure 22, in hBMECs, P-rHDL exhibited a similar intracellular delivery efficiency to rHDL. As shown in Figure 23, a quantitative analysis based on fluorescence image analysis confirmed no significant difference. As shown in Figure 24, in iBMECs, P-rHDL exhibited a similar intracellular delivery efficiency to rHDL. As shown in Figure 25, a quantitative analysis based on fluorescence image analysis confirmed no significant difference. Therefore, since P-rHDL showed similar delivery efficiency to rHDL in BMEC, it can be seen that P-rHDL can have the same delivery effect as rHDL functionally without being affected by PEGylation.

### [Example 6]

### Confirmation of blood-brain barrier permeation efficiency of P-rHDL

In order to confirm the blood-brain barrier permeability of P-rHDL, a monolayer of hBMECs was formed in the insert of a transwell. It was treated with DiD fluorescently labeled P-rHDL and rHDL, respectively. The permeation efficiency was compared with that of the control groups (fluorescein, rhodamine B, and FITC-dextran (4, 70, and 150 kDa)) at time points (4 and 24 hours). The permeation of the control group, rHDL, and P-rHDL was taken from the bottom well of the transwell, and fluorescence was measured using a microplate reader to compare the permeability.

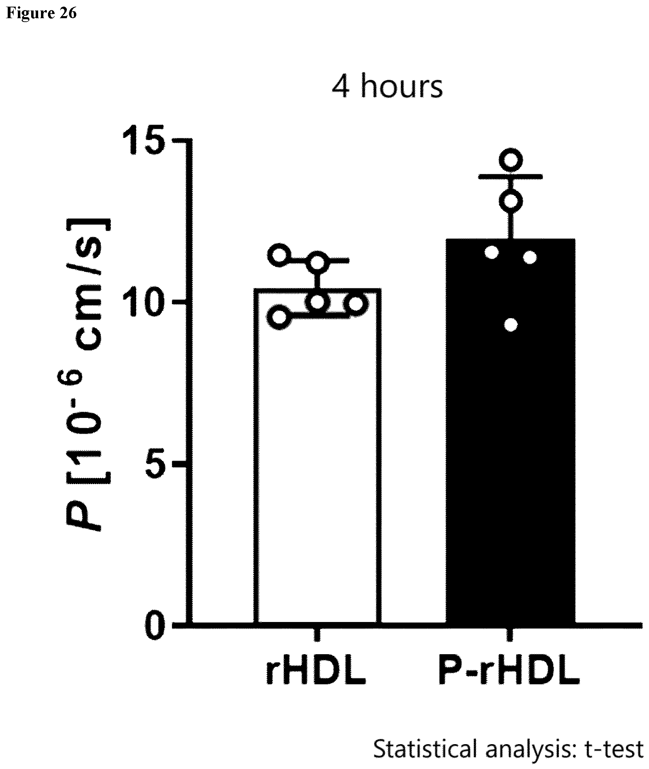

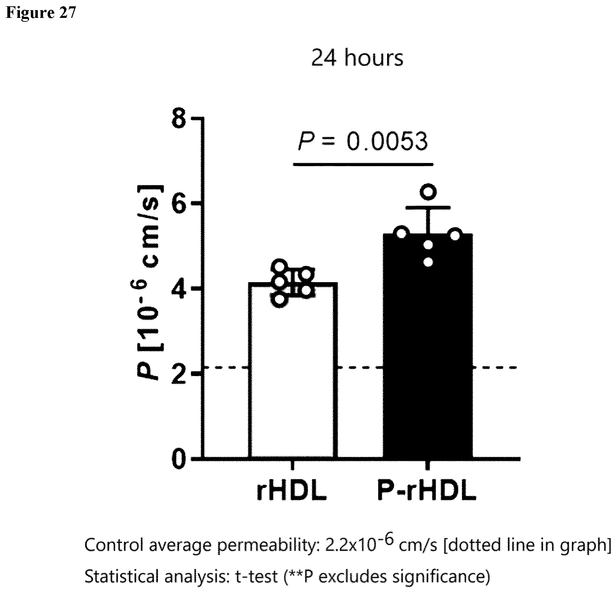

As shown in Figure 26, at 4 hours, the initial permeation time, P-rHDL exhibited similar blood-brain barrier permeation efficiency compared to rHDL, with no significant difference. As shown in Figure 27, at 24 hours, P-rHDL exhibited a statistically significant difference in blood-brain barrier permeability (*p* = 0.0053) compared to rHDL. However, this difference was excluded from significance because P-rHDL and rHDL exhibited similar levels of cell delivery efficiency, and PEGylation did not appear to contribute to improved permeation efficiency. Therefore, P-rHDL exhibited similar blood-brain barrier permeation efficiency to rHDL up to 24 hours. Comparison with the control group confirmed that both rHDL and P-rHDL exhibited more than twice the permeation efficiency, demonstrating a significant difference.

## Claims

1. A PEGylated reconstituted high-density lipoprotein (rHDL) nanoparticle comprising a PEGylated lipid, a phospholipid, and apolipoprotein E, wherein the synthetic mixing molar ratio of the apolipoprotein E and the PEGylated lipid is 1:0.5 to 1:50.

2. The PEGylated reconstituted high-density lipoprotein (rHDL) nanoparticle according to claim 1, wherein the synthetic mixing molar ratio of the apolipoprotein E and the PEGylated lipid is 1:1 to 1:10.

3. The PEGylated reconstituted high-density lipoprotein (rHDL) nanoparticle according to claim 1, wherein the synthetic mixing molar ratio of the apolipoprotein E and the PEGylated lipid is 1:1 to 1:5.

4. The PEGylated reconstituted high-density lipoprotein (rHDL) nanoparticle according to claim 1, wherein the average molecular weight of the polyethylene glycol in the PEGylated lipid is 550 to 5,000.

5. The PEGylated reconstituted high-density lipoprotein (rHDL) nanoparticle according to claim 1, wherein the average molecular weight of the polyethylene glycol in the PEGylated lipid is 1,000 to 2,000.

6. The PEGylated reconstituted high-density lipoprotein (rHDL) nanoparticle according to claim 1, wherein the PEGylated reconstituted high-density lipoprotein (rHDL) nanoparticle has a size of 5 to 50 nm.

7. The PEGylated reconstituted high-density lipoprotein (rHDL) nanoparticle according to claim 1, wherein the PEGylated reconstituted high-density lipoprotein (rHDL) nanoparticle has a size of 10 to 35 nm.

8. The PEGylated reconstituted high-density lipoprotein (rHDL) nanoparticle according to claim 1, wherein the apolipoprotein E is apolipoprotein E2, E3, or E2 and E3.

9. The PEGylated reconstituted high-density lipoprotein (rHDL) nanoparticle according to claim 1, wherein the PEGylated reconstituted high-density lipoprotein (rHDL) nanoparticle may further comprise apolipoprotein A1.

10. The PEGylated reconstituted high-density lipoprotein (rHDL) nanoparticle according to claim 1, wherein the phospholipid is at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-amino-propyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DCChol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA.

11. A composition for preventing or treating neurodegenerative diseases, comprising the PEGylated reconstituted high-density lipoprotein nanoparticles according to any one of claims 1 to 10.

12. The composition for preventing or treating neurodegenerative diseases according to claim 11, wherein the neurodegenerative disease is selected from the group consisting of Parkinson's disease, Alzheimer's disease, Pick's disease, Huntington's disease, Creutzfeldt-Jakob disease, Lou Gehrig's disease, spinocerebellar degeneration, spinocerebellar ataxia, prion disease, cognitive dysfunction, senile dementia, dementia with Lewy bodies, frontotemporal dementia, vascular dementia, alcoholic dementia, presenile dementia, Machado-Joseph disease, dystonia, multiple system atrophy, progressive supranuclear palsy, Friedreich's ataxia, and temporal lobe epilepsy.

13. The composition for preventing or treating neurodegenerative diseases according to claim 11, wherein the neurodegenerative disease is Alzheimer's disease.

14. A method for producing PEGylated reconstituted high-density lipoprotein (rHDL) nanoparticles comprising a PEGylated lipid, a phospholipid, and apolipoprotein E, wherein the method comprises:
a step of injecting a phospholipid and PEGylated lipid solution into a second inlet located in the center of a microfluidic device comprising three inlets and one outlet, and
a step of injecting high-density lipoprotein (HDL) or reconstituted high-density lipoprotein (rHDL) comprising an apolipoprotein and a phospholipid into the first and third inlets located on either side.
